# EUROPÄISCHE PATENTSCHRIFT

(11) **EP 1 715 815 B1**
(45) Veröffentlichungstag und Bekanntmachung des Hinweises auf die Patenterteilung: **24.04.2013**
(21) Anmeldenummer: 05700374.1
(22) Anmeldetag: 21.02.2005
(51) Int. Cl.: A61F 2/36

(54) **PROTHESE ZUM OBERFLÄCHENERSATZ IM BEREICH DER KUGEL VON KUGELGELENKEN**
PROSTHESIS FOR REPLACING THE SURFACE IN THE AREA OF THE BALL OF BALL-AND-SOCKET JOINTS
PROTHESE POUR UN REMPLACEMENT EN SURFACE DANS LA ZONE DE LA SPHERE D'UNE ENARTHROSE

(30) Priorität: 20.02.2004 EP 04003943
(43) Veröffentlichungstag der Anmeldung: 02.11.2006
(73) Patentinhaber: Synthes GmbH, 4436 Oberdorf (CH)
(72) Erfinder: KROPF, Philipp, CH-6330 Cham (CH); HERTEL, Ralph, CH-3074 Muri (CH)
(74) Vertreter: Lusuardi, Werther
(86) Internationale Anmeldenummer: PCT/CH2005/000099
(87) Internationale Veröffentlichungsnummer: WO 2005/079709

(56) Entgegenhaltungen:
- DE-A1- 2 751 537
- FR-A- 2 578 739
- FR-A- 2 737 970
- US-A- 4 328 593
- US-A- 5 549 704
- US-A- 6 048 365

## Beschreibung

Die Erfindung betrifft eine Prothese gemäss Oberbegriff des Anspruch 1, einen Materialsatz zur Herstellung einer besonderen Ausführung einer solchen Prothese gemäss Anspruch 13 sowie einen Satz von Prothesen unterschiedlicher Grösse gemäss Anspruch 14.

Prothesen dieser Art werden bei Kugelgelenken, insbesondere bei Schulter- und Hüftgelenk, eingesetzt. Grundsätzlich gibt es bei Prothesen für Kugelgelenke zwei unterschiedliche Lösungsvarianten.

Bei einer ersten Variante wird die Kugel ganz oder zumindest zur Hälfte abgetrennt und durch eine Prothese ersetzt, wobei diese einen Schaft aufweist, der in den verbleibenden Knochen bzw. dessen Markraum eingeführt wird. Derartige Schaftprothesen eignen sich besonders für ältere Menschen und/oder im Fall von Knochenbrüchen, wie z.B. Oberschenkelhalsbrüchen.

Bei einer zweiten Variante wird lediglich die Oberfläche des Knochens bzw. Knorpels zumindest teilweise ersetzt. Man spricht in diesem Fall von "Oberflächenersatzprothesen", "Resurfacing-Prothesen" oder auch "Kappenprothesen". Entsprechende Prothesen werden z.B. unter den Warenzeichen "Durom" und "Copeland" angeboten. Diese Prothesenart hat den Vorteil, dass ihre Implantation minimal invasiv ist. Die Operationsdauer ist kürzer. Bei einem Ersatz der Prothese gibt es mehr Möglichkeiten. Insbesondere bietet sich eine Rückzugsoperation an, bei der die Prothese durch eine Schaftprothese, wie sie oben beschrieben ist, ersetzt wird.

Ein Problem bei Oberflächenersatzprothesen besteht jedoch darin, dass ihre Befestigung meist schwieriger ist als bei Prothesen mit Schaft. Es gibt verschiedene Ansätze die Befestigung zu realisieren.

So gibt es Oberflächenersatzprothesen, welche nur aus einer Kappe bestehen, dafür aber im Wesentlichen die gesamte Gelenkkugel umschliessen. Dies führt zumindest dem Anschein nach zu einer besseren Verteilung der Kräfte, es muss jedoch mehr Knochenmaterial abgetragen werden und die Stabilität bei Zugkräften ist unter Umständen unvollkommen.

Ferner gibt es Oberflächenprothesen, die einen Stiel aufweisen, wobei dessen Länge in etwa dem Kugeldurchmesser entspricht. Dadurch werden die Kräfte auf zusätzliche Kontaktflächen verteilt und die Prothese ist auf der Gelenkkugel besser zentriert. Die Lösung ist jedoch unvollkommen, unter anderem weil der Stiel zu Hebelkräften und damit unter Umständen zu einer Lockerung der Prothese führen kann.

In einigen Fällen stellt sich bei den bekannten Prothesen auch das Problem, dass der Knochen im Bereich des Gelenkkopfes und damit insbesondere bei den Kontaktflächen mit der Prothese abstirbt.

US-A-4,328,593 zeigt eine Oberflächenprothese mit einer Kappe, die grösser als eine Halbkugel ist und einen zylindrischen, hohlen Stiel aufweist, der weit über den unteren Rand der Kappe herausragt. US-A-5,549,704 zeigt eine zum genannten US-Dokument ähnliche Oberflächenprothese. FR-A-2 578 739 zeigt eine Oberflächenprothese, bei der ein Teil einer Kappe kleiner als eine Halbkugel ist und ein Laschenteil über die Kappe vorsteht. Zur Befestigung ist ein Gewindebolzen vorgesehen, in den ein Zapfen der Kappe eingreift. FR-A-2 737 970 zeigt eine Oberflächenprothese mit einer Kappe, die kleiner oder grösser als eine Halbkugel ist und die einen mittigen Stiel aufweist.

### Darstellung der Erfindung

Es stellt sich daher die Aufgabe, eine Prothese der eingangs genannten Art bereitzustellen, die die oben genannten Nachteile zumindest teilweise vermeidet.

Diese Aufgabe wird mit den Merkmalen von Anspruch 1 gelöst.

Eine derartige Prothese hat den Vorteil, dass ihre Befestigung stabil ist und aufgrund der grossen Auflagefläche die zwischen Knochen und Prothese wirkenden Kräfte gut verteilt sind. Ferner greift die Krone bei geeigneter Anordnung und Dimensionierung direkt in die tragenden und intakten Strukturen des Knochens womit eine langfristig gute Stabilität erreicht wird. Die Implantation der Prothese ist wenig invasiv. Der Kugelschalenabschnitt ist mit einer Höhe h kleiner oder höchstens gleich dem Radius der Kugel und somit kleiner oder höchstens gleich einer halbkugeligen Kugelschale. Bevorzugterweise beträgt die Höhe h zwischen 70-85 % des Kugelradius. Dies reicht um die knorpelig überzogene Artikulationsfläche abzudecken. Eine Kugel die sich schliesst, kann ab der sich verengenden Stelle nicht mehr bearbeitet werden. Die Krone ist im wesentlichen gleich dem Kugelabschnitt bzw. endet in dessen Durchmesserebene und ist gegebenenfalls sogar kürzer. Dies reicht für die Verankerung im Knochen. Bei einem Zweiteingriff als Revision kann mit einer Säge bündig am sichtbaren Kugelabschnitt abgesägt werden. Das heisst beim Absägen ist die Schnittführung sichtbar und ein schwieriges Ausschneiden oder Ausreissen einer langen Krone ist nicht nötigt.

Dadurch, dass der Kugelabschnitt kleiner/gleich als der Kugelradius ist, kann die Krone weit aussen positioniert werden. So wird die Kronenoberfläche maximiert und die (belastete) Kontaktfläche zum Knochen vergrössert und somit die Stabilität erhöht.

Bevorzugterweise wächst die Krone proportional zum Implantat (Proportionalität, d.h. je grösser der Kugeldurchmesser, desto grösser die Krone). Dies entspricht der Anatomie und bewirkt eine besonders gute Stabilität der Befestigung der Prothese.

Bei der erfindungsgemässen Prothese muss nur wenig Knochenmaterial entfernt werden und es bleibt damit viel Knochen erhalten. Die Operationsdauer ist relativ kurz.

Bevorzugterweise ist die Kronenwandung nicht überall gleich dick, sondern sie verläuft konisch und nimmt Richtung Verbindungsstelle zu. Dadurch wird beim Einbringen ein gewisser, kontrollierter Druck aufgebaut, der die Stabilität verbessert. Weiter sind bevorzugt eine bis drei Seiten der Krone parallel, 1-3 Seiten konisch. Je nach Anordnung wird der Druck kontrolliert in eine oder mehrere Richtungen geführt. Bei einer anderen bevorzugten Ausführung sind die Innenseiten der Krone parallel und die Aussenseiten verlaufen konisch. Dadurch wird der Druck, der beim Einführen entsteht, nach aussen, Richtung Innenseite des Kugelabschnittes, aufgebaut. Der Knochenabschnitt wird zwischen Krone und Kugelabschnittinnenseite geklemmt. Weiter ist es bevorzugt, die Krone mit Löchern zu versehen. So kann der durchblutete Knochen im Innern der Krone "überleben". Es wird nur ein ringförmiges Segment Knochen entfernt, welches durch die Krone mit Löchern ersetzt wird. Durch die Löcher im Ring können die Knochensegmente wieder zueinander verwachsen. Bevorzugterweise folgt nach dem zylindrischen Teil ein konischer Teil mit umfänglichen Einstichen (kein Gewinde), die Stabilität gegen ein Ausreissen bieten. Sie wirken wie Widerhaken.

Die genannten Vorteile ergeben sich auch bei den Gegenständen der weiteren unabhängigen Ansprüche

### Kurze Beschreibung der Zeichnungen

Weitere Vorteile und bevorzugte Ausführungsbeispiele ergeben sich aus den abhängigen Ansprüchen, sowie aus der nun folgenden Beschreibung anhand der Figuren. Dabei zeigen:
Fig. 1 einen Schnitt durch eine bevorzugte Ausführung der erfindungsgemässen Prothese;
Fig. 2 eine perspektivische Ansicht der bevorzugten Ausführung der erfindungsgemässen Prothese von Fig. 1;
Figur 3 einen Satz von Prothesen;
Figur 4 eine Ausführungsform in Schnittdarstellung; und
Figur 5 die Ausführung von Figur 4 in schaubildlicher Darstellung

### Wege zur Ausführung der Erfindung

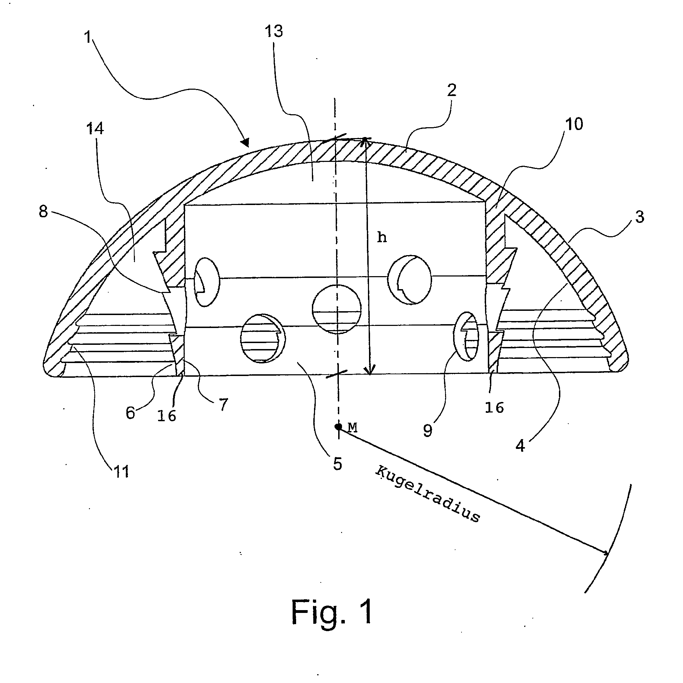

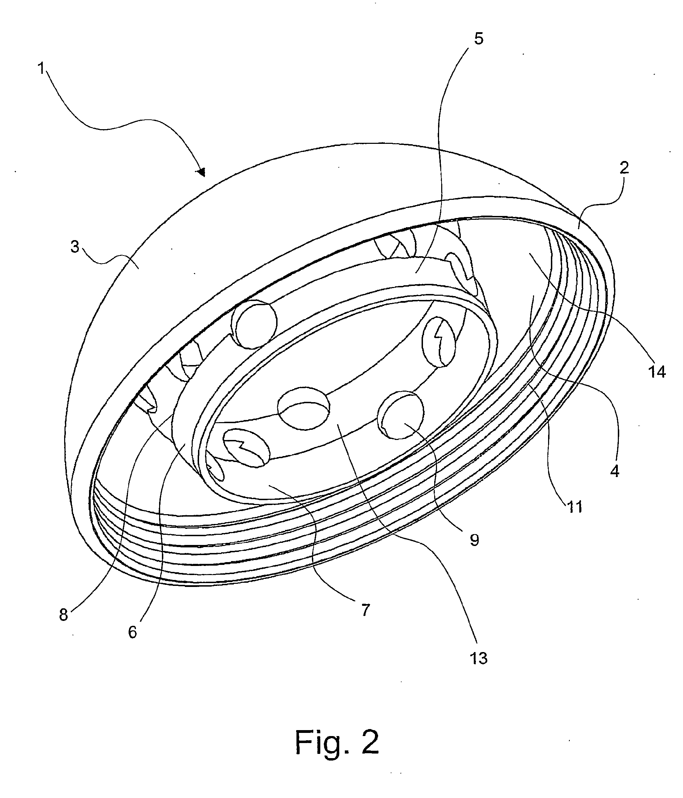

Fig. 1 zeigt einen Schnitt durch eine bevorzugte Ausführung der erfindungsgemässen Prothese. Fig. 2 zeigt eine perspektivische Ansicht derselben Prothese. Die Prothese dient zum teilweisen Ersatz der Oberfläche einer Kugel eines Kugelgelenkes, wie z.B. dem Oberschenkelkopf. Die Prothese 1 besteht im Wesentlichen aus einem Kugelschalenausschnitt bzw. Kugelschalenabschnitt 2 und einer darin angeordneten Krone 5.

Der Kugelschalenausschnitt 2 hat die Form einer Halbkugel oder bevorzugt die Form eines Ausschnittes einer Halbkugel. Dies hat den Vorteil, dass bei der Implantation nur eine partielle Resektion der Gelenkkugeloberfläche vorgenommen werden muss. Der Kugelschalenausschnitt 2 ist bevorzugt also etwas kleiner als eine Halbkugel, d.h. er erstreckt sich vorzugsweise über einen Winkel von 145 bis 180 Grad. Der Kugelschalenausschnitt 2 kann auch als "Kappe" oder, wenn er wie in der gezeigten Ausführung im Wesentlichen halbkugelförmig ist, als "Hemisphäre" bezeichnet werden. Insbesondere bevorzugt ist eine Kappe, also ein Kugelschalenausschnitt, der kleiner als eine Halbkugel ist und insbesondere eine Höhe h von nur 65 % - 90 % und insbesondere 70 % - 85 % und insbesondere ca. 80 % des Kugelradius aufweist, entsprechend einem Winkel vom Mittelpunkt M aus von ca. 117 bis 162 Grad bzw. 126 bis 153 Grad, bzw. ca. 144 Grad, wenn eine Halbkugel 180° entspricht. In Figur 1 ist die Höhe h des Kugelschalenabschnittes bezeichnet sowie der Mittelpunkt M der Kugel.

Die Aussenfläche 3 des Kugelschalenausschnittes 2 ist zum Aufliegen in einer Gelenkpfanne ausgestaltet. Die Aussenfläche 3 ist insbesondere glatt, so dass sie sich in der Gelenkpfanne gut bewegen kann. Bei der Gelenkpfanne kann es sich ebenfalls um eine Prothese handeln. Auf der Innenfläche 4 ist eine Riffelung 11 vorgesehen, welche durch im Bereich des Randes verlaufende Ringnuten gebildet ist. Eine Ausgestaltung des Kugelschalenausschnittes 2 mit einem derartigen oder anders gearteten Relief führt zu einem besseren Halt der Prothese.

Die Krone 5 dient zur Befestigung bzw. Verankerung der Prothese am bzw. im Knochen und/oder zur besseren Verteilung der Auflagekräfte. Sie hat bevorzugt im Wesentlichen die Form einer Zylindermantelfläche. Ihr Durchmesser beträgt 45 bis 75 Prozent, insbesondere 55 bis 65 Prozent, insbesondere etwa 60 Prozent, des Durchmessers des Kugelschalenausschnittes 2. Durch diese Dimensionierung greift die Krone 5 in bzw. an einen sehr stabilen Teil des Knochens, wobei dessen röhrenartige Struktur berücksichtigt wird. Die Krone 5 weist eine Aussenfläche 6 und eine Innenfläche 7 auf. Auf der Aussenfläche 6 ist eine Riffelung 8 in Form von um die Krone 5 herumführenden Ringwülsten vorgesehen. Die Riffelung kann auch auf der Innenfläche 7 oder auf beiden Flächen 6, 7 vorgesehen sein. Eine Ausgestaltung der Krone 5 mit einem derartigen oder anders gearteten Relief führt zu einem besseren Halt der Prothese. Die Krone weist an ihrem freien Ende bevorzugt einen kurzen zylindrischen Abschnitt 16 (Einführung) auf, die das Einführen des Implantats in die Ringnut im Knochen erleichtert. Die Krone 5 ist zumindest teilweise im Kugelschalenausschnitt 2 angeordnet. Durch die Krone 5 ist der vom der Kugelschalenausschnitt 2 zur Aufnahme eines Knochenendes gebildete offene Hohlraum im Wesentlichen in einen inneren, ersten Hohlraum 13 und einen äusseren, ringförmig darum herum angeordneten, zweiten Hohlraum 14 unterteilt. Der Ausdruck "im Wesentlichen" soll in diesem Zusammenhang anzeigen, dass die Krone 5, wie weiter unten näher erläutert, Aussparungen aufweisen kann, welche die beiden Hohlräume 13 und 14 verbinden. Die Hohlräume 13, 14 dienen der Aufnahme des Knochenendes und sind daher auf der dem Knochen zugewandten Seite offen. Die Öffnung des ersten Hohlraums 13 ist kreisflächenförmig, die des zweiten Hohlraumes 14 ist ringförmig. Im Falle der implantierten Prothese steht der Knochen in diese Hohlräume 13, 14 hinein. Insbesondere nach einer Verwachsungsphase sind die Hohlräume im Wesentlichen ganz mit Knochen oder knochenähnlichem Gewebe ausgefüllt. In der Krone 5 sind bevorzugt ca. fünf bis zehn Aussparungen 9, insbesondere im Wesentlichen runde Löcher, vorgesehen. Durch diese kann der in den beiden Hohlräumen 13, 14 befindliche Knochen hindurch und zusammenwachsen. Dies ermöglicht eine bessere Durchblutung des Knochens und führt zu einem besseren Halt der Prothese 1, insbesondere auch bei Zugkräften. Die Aussparungen können sich auch über die ganze Länge der Krone erstrecken, so dass die Krone durch Spalte unterbrochen ist und/oder in einzelne Zacken unterteilt ist. Die Innenfläche 4 des Kugelschalenausschnittes 2 und die Oberfläche, d.h. die Aussenfläche 6 und Innenfläche 7, der Krone 5 sind für einen Kontakt und/oder ein Verwachsen mit dem Knochen ausgestaltet. Die Flächen sind hierfür insbesondere rauh.

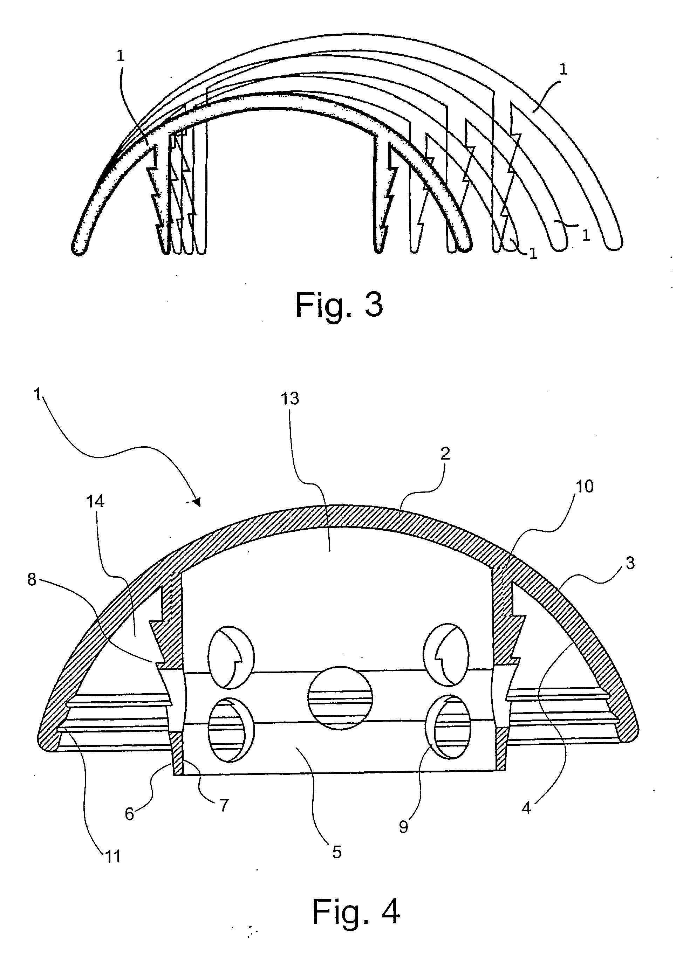

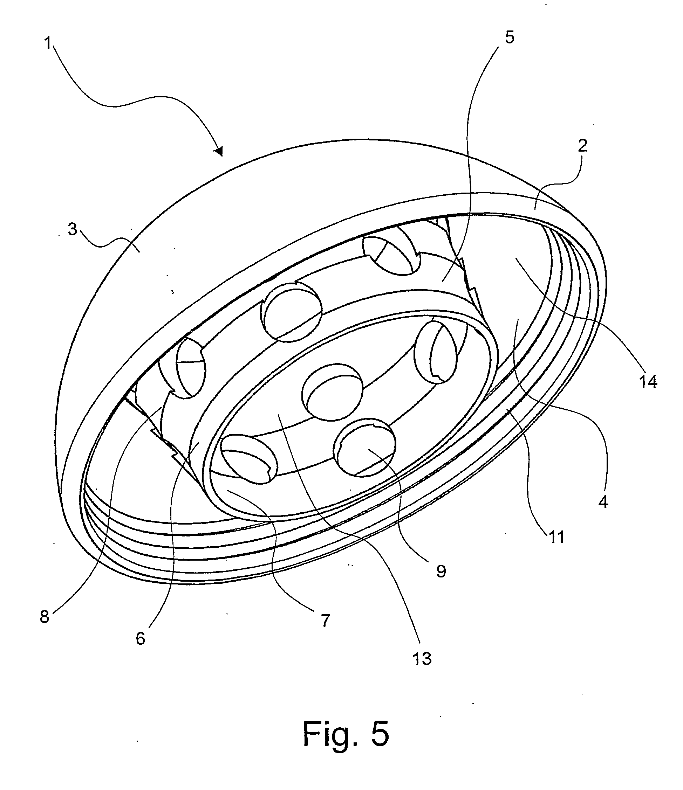

Die Krone 5 ist bevorzugt mit dem Kugelschalenausschnitt 2 zusammen einstückig ausgestaltet. Krone 5 und Kugelschalenausschnitt 2 können jedoch auch zunächst getrennt gefertigt und anschliessend an einer Verbindungsstelle 10 verschweisst sein. Die Krone 5 ist koaxial zum kreisförmigen Rand des Kugelschalenausschnittes 2 angeordnet, d.h. die Symmetrieachsen von Krone 5 und Kugelschalenausschnitt 2 liegen auf derselben Gerade. Der Rand der Krone 5 verläuft bevorzugt im Wesentlichen in derselben Ebene wie der Rand des Kugelschalenausschnittes 2 (Figuren 1 und 2). Der Kronenrand verläuft bei dieser Ausgestaltung in der Ebene oder steht um ein Weniges, z.B. um 1 bis 3 mm, über die Ebene hinaus, in welcher der Rand des Kugelschalenausschnittes 2 verläuft, was in Figur 4 und 5 dargestellt ist. So ist sichergestellt, dass beim Aufsetzen der Prothese die Krone 5 den vorbereiteten Knochen zuerst kontaktiert und eine für die Krone 5 vorbereitete Nut als Ausrichtungs- bzw. Zentrierhilfe verwendet werden kann. Die Ausgestaltung mit einer nicht wesentlich, also z.B. nur 0,2 bis 5 mm oder 1-3 mm, über den Rand des Kugelschalenausschnittes 2 hinausstehenden Krone 5 hat den Vorteil, dass, falls ein Ersatz der Prothese durch eine Schaftprothese notwendig ist, die bestehende Prothese nicht aus dem Knochen herausgelöst werden muss, sondern einfach zusammen mit einem Teil des Knochens abgesägt werden kann. Es muss dabei nur Knochen durchsägt werden. Der knochenseitige Teil der Gelenkkugel bleibt in einem für eine Schaftprothese ausreichendem Masse stehen. Bei einer weiteren Ausführungsform ist die Krone kürzer, z.B. ebenfalls 1-3 mm oder 0,2 bis 5 mm, als der Kugelabschnitt und erreicht somit die Ebene des Kugelabschnittrandes nicht.

Die erfindungsgemässe Prothese hat den Vorteil, dass sie konsequent die erforderliche Knochenresektion minimiert. Dies vergrössert insbesondere die Auswahl möglicher Rückzugsoperationen.

Bei der Implantation der erfindungsgemässen Prothese wird zunächst der Kopf des Knochens mit einem Spezialwerkzeug entsprechend der Form des Kugelschalenausschnittes abgeschält und/oder abgefräst. Anschliessend wird eine Ringnut in den Knochen geschnitten, welche auf die Abmessungen der Krone abgestimmt ist. Dies erfolgt bevorzugt mit einem eigens dafür vorgesehenen Kronenfräser. Dann wird die Prothese auf den derart vorbereiteten Knochen aufgedrückt und/oder in diesen eingeschlagen. Es ist kein Zement erforderlich. Die typische Operationszeit kann bei Verwendung der erfindungsgemässen Prothese signifikant reduziert werden.

Die erfindungsgemässe Prothese kann für Kugelgelenke allgemein verwendet werden, insbesondere jedoch für das Schulter und das Hüftgelenk. Die Prothese wird hierfür in verschiedenen Grössen bereitgestellt. Die Prothese wird ferner in verschiedenen Grössen bereitgestellt, µm eine auf die Anatomie bzw. die Körpermasse des Patienten optimal abgestimmte Prothese aussuchen zu können.

In einer weiteren Ausführung der Erfindung werden Kugelschalenausschnitt und Krone separat, d.h. als Einzelteile bzw. als Materialsatz von Einzelteilen bereitgestellt. Am Kugelschalenausschnitt und/oder an der Krone ist dabei ein mechanisches Befestigungsmittel wie z.B. ein Schraubgewinde, ein Bajonettverschluss oder eine Klemmvorrichtung vorgesehen. Mittels dieser lassen sich Kugelschalenausschnitt und Krone verbinden. Bei einer solchen Ausführung als Materialsatz ist es möglich verschieden ausgestaltete Kugelschalen, insbesondere mit verschiedenen Kugelradien, und verschieden ausgestaltete Kronen, insbesondere mit verschiedenen Längen und Durchmessern, in Abstimmung auf die Anatomie des Patienten miteinander zu kombinieren. Es können so, insbesondere während der Operation, speziell angepasste Prothesen hergestellt werden.

In einer weiteren Ausführung der Erfindung ist die Prothese statt zum Einschlagen bzw. Aufdrücken zum Ein- bzw. Aufschrauben ausgestaltet. Die Riffelung auf der Krone und/oder die Riffelung auf der Innenfläche des Kugelschalenausschnittes ist hierfür als Gewinde ausgebildet. Ferner weist die Prothese insbesondere Kerben und/oder Abflachungen als Angriffsfläche für ein Drehwerkzeug auf.

Bei der in den Figuren gezeigten Ausführung hat die Krone im Wesentlichen die Form einer Zylindermantelfläche. Die Krone, bzw. ihre Innen- und Aussenfläche, kann jedoch auch ganz oder teilweise konisch ausgestaltet werden. In einer möglichen Variante ist die Innenfläche im Wesentlichen zylindrisch und die Aussenfläche im Wesentlichen konisch. In einer weiteren Variante ist die Innenfläche im Wesentlichen konisch und die Aussenfläche im Wesentlichen zylindrisch. In einer weiteren Variante sind sowohl die Innenfläche als auch die Aussenfläche im wesentlichen konisch. Die Konizität ist dabei bevorzugt so, dass die Wandstärke der Krone zum Kugelschalenausschnitt hin zunimmt, z.B. von 1 mm am Rand bis auf 4 mm am Übergang zum Kugelschalenausschnitt. Dies hat den Vorteil, dass beim Aufsetzen der Prothese zusätzlicher Druck erzeugt wird, was den Halt der Prothese auf dem Knochen verbessert.

Ferner hat bei der in den Figuren gezeigten Ausführung die Krone den Grundriss eines Kreises. Der Grundriss kann jedoch auch anders gewählt werden, z.B. sternförmig oder in Form eines insbesondere gleichmässigen Vielecks, z.B. eines Sechsecks.

Wie vorstehend erwähnt kann der Durchmesser der Krone einen Prozentsatz des Durchmessers der Kugelschalenabschnittes betragen. Diesfalls ist ein Wert von grösser als 50 % besonders bevorzugt und insbesondere 55 % bis 75 %, insbesondere 55 % bis 65 % und insbesondere 60 %. Bei einem Satz von von erfindungsgemässen Prothesen verschiedener, zunehmender Grösse wächst also der Durchmesser der Krone mit, da er grössenmässig an den Durchmesser des Kugelschalenabschnittes gekoppelt ist. Dies ist in Figur 3 schematisch dargestellt, worin ein Satz von vier Prothesen 1 verschiedener Grösse in einer Art Schnittdarstellung und mit allen vier Prothesen in der selben Figur dargestellt ist, bei denen der Kronendurchmesser jeweils ca. 60 % des Durchmessers (gemessen aussen am freien Rand) des Kugelabschnittes beträgt.

Während anhand der Figuren im Wesentlichen nur eine bevorzugte Ausführung der erfindungsgemässen Prothese beschrieben wurde ist es dem Fachmann klar, dass die meisten der aufgeführten Merkmale unter Berücksichtigung ihrer Funktion variiert, anders kombiniert oder weggelassen werden können.

## Patentansprüche

1. Prothese zum Oberflächenersatz im Bereich einer Kugel von Kugelgelenken, wobei die Prothese (1) einen Kugelschalenausschnitt (2) mit einer zum Aufliegen in einer Gelenkpfanne ausgestalteten Aussenfläche (3) aufweist, und zur Befestigung und/oder zur besseren Verteilung von Auflagekräften eine Krone (5) aufweist, welche einen vom Kugelschalenausschnitt (2) zur Aufnahme eines Knochenendes gebildeten Hohlraum im Wesentlichen in einen ersten Hohlraum (13) und einen zweiten Hohlraum (14) unterteilt und wobei der Kugelschalenausschnitt (2) im Wesentlichen eine Form einer Halbkugel oder bevorzugterweise eines Ausschnittes einer Halbkugel hat, **dadurch gekennzeichnet dass** der freie Rand der Krone (5) im Wesentlichen in derselben Ebene verläuft wie der freie Rand des Kugelschalenausschnittes (2).

2. Prothese nach Anspruch 1, **dadurch gekennzeichnet, dass** der Kugelschalenausschnitt eine Höhe h aufweist, die 65 % bis 90 % des Kugelradius beträgt und insbesondere 70 % bis 85 % und insbesondere ca. 80 % des Kugelradius beträgt.

3. Prothese gemäss einem der vorhergehenden Ansprüche, **dadurch gekennzeichnet, dass** ein Durchmesser der Krone (5) 45 bis 75 Prozent, insbesondere 55 bis 65 Prozent und insbesondere etwa 60 Prozent, eines Durchmessers des Kugelschalenausschnittes (2) beträgt.

4. Prothese gemäss einem der vorhergehenden Ansprüche, **dadurch gekennzeichnet, dass** eine Innenfläche (4) des Kugelschalenausschnittes (2) und/oder eine Oberfläche der Krone (5) für einen Kontakt und/oder ein Verwachsen mit einem Knochen ausgestaltet ist und hierfür insbesondere rauh ist.

5. Prothese gemäss einem der vorhergehenden Ansprüche, **dadurch gekennzeichnet, dass** die Krone (5) mindestens eine Aussparung (9) aufweist, insbesondere fünf bis zehn Aussparungen (9).

6. Prothese gemäss einem der vorhergehenden Ansprüche, **dadurch gekennzeichnet, dass** die Krone (5) auf einer Aussenfläche (6) und/oder Innenfläche (7) ein Relief aufweist, insbesondere eine Riffelung (8), welche aus um die Krone (5) herumführende Ringwülsten gebildet ist.

7. Prothese gemäss einem der vorhergehenden Ansprüche, **dadurch gekennzeichnet, dass** der Kugelschalenausschnitt (2) auf einer Innenfläche (4) ein Relief aufweist, insbesondere eine Riffelung, welche entlang eines Randes des Kugelschalenausschnitts (2) verläuft.

8. Prothese gemäss einem der vorhergehenden Ansprüche, **dadurch gekennzeichnet, dass** sie zum Auf- bzw. Einschrauben ausgestaltet ist, wobei die Krone (5) und/oder eine Innenfläche (4) des Kugelschalenausschnittes (2) ein Gewinde aufweist und die Prothese (1) insbesondere Kerben und/oder Abflachungen als Angriffsfläche für ein Drehwerkzeug aufweist.

9. Prothese gemäss einem der vorhergehenden Ansprüche, **dadurch gekennzeichnet, dass** ein Grundriss der Krone (5) eine Form eines Kreises oder eines im wesentlichen gleichmässigen Vieleckes hat und insbesondere, dass der freie Rand einen im wesentlichen zylindrischen Abschnitt (16) aufweist.

10. Prothese gemäß Anspruch 9, **dadurch gekennzeichnet, dass** die Krone (5) im Wesentlichen koaxial zu einem Rand von Kugelschalenausschnitt (2) angeordnet ist.

11. Prothese gemäss einem der vorhergehenden Ansprüche, **dadurch gekennzeichnet, dass** Kugelschalenausschnitt (2) und Krone (5) zusammen einstückig ausgebildet sind und/oder miteinander verschweisst sind.

12. Prothese gemäss einem der vorhergehenden Ansprüche **dadurch gekennzeichnet, dass** Kugelschalenausschnitt (2) und Krone (5) als Einzelteile ausgestaltet sind und mit einem mechanischen Befestigungsmittel, insbesondere mit einem Schraubgewinde, einem Bajonettverschluss oder einer Klemmvorrichtung, miteinander verbun-, den sind.

13. Materialsatz zur Herstellung einer Prothese gemäss Anspruch 12, **dadurch gekennzeichnet, dass** er verschiedenartige Kugelschalenausschnitte (2) gemäss Anspruch 12 und verschiedenartige Kronen (5) gemäss Anspruch 12 umf asst, wobei die Kugelschalenausschnitte (2) und die Kronen (5) zur Schaffung speziell angepasster Prothesen (1) in verschiedenen Kombinationen jeweils paarweise aneinander befestigbar sind.

14. Satz von Prothesen nach einem der Ansprüche 1 bis 12, welcher Satz von Prothesen mit Kugelschalenausschnitten von voneinander verschiedenen Durchmessern, aber insbesondere gleichem Verhältnis der Höhe h des Kugelschalenausschnittes zum jeweiligen Kugeldurchmesser gebildet sind, bei welchen der Durchmesser der Krone jeweils im wesentlichen den selben Prozentsatz des Durchmessers des Kugelschalenausschnittes beträgt.

## Claims

1. A prosthesis for surface replacement in the region of a ball of ball-and-socket joints, wherein the prosthesis (1) has a spherical calotte (2) having an outer surface (3) which is designed for resting in a joint socket, and, for fixing purposes and/or for better distribution of contact forces, has a crown (5) which essentially divides a hollow space formed by the spherical calotte (2) for accommodating a bone end into a first hollow space (13) and a second hollow space (14), and wherein the spherical calotte (2) substantially has the form of a hemisphere or preferably a section of a hemisphere, **characterized in that** the free edge of the crown (5) runs substantially in the same plane as the free edge of the spherical calotte (2).

2. The prosthesis as claimed in claim 1, **characterized in that** the spherical calotte has a height h which is 65% to 90% of the ball radius and in particular 70% to 85% and in particular approx. 80% of the ball radius.

3. The prosthesis as claimed in one of the preceding claims, **characterized in that** a diameter of the crown (5) is 45 to 75%, in particular 55 to 65% and in particular about 60% of a diameter of the spherical calotte (2).

4. The prosthesis as claimed in one of the preceding claims, **characterized in that** an inner surface (4) of the spherical calotte (2) and/or a surface of the crown (5) is designed for making contact and/or coalescing with a bone and in particular is rough for this purpose.

5. The prosthesis as claimed in one of the preceding claims, **characterized in that** the crown (5) has at least one recess (9), in particular five to ten recesses (9).

6. The prosthesis as claimed in one of the preceding claims, **characterized in that** the crown (5) has a relief on an outer surface (6) and/or inner surface (7), in particular a corrugation (8) which is formed from annular beads running around the crown (5).

7. The prosthesis as claimed in one of the preceding claims, **characterized in that** the spherical calotte (2) has a relief on an inner surface (4), in particular a corrugation which runs along an edge of the spherical calotte (2).

8. The prosthesis as claimed in one of the preceding claims, **characterized in that** it is designed for screwing on or in, wherein the crown (5) and/or an inner surface (4) of the spherical calotte (2) has a thread and the prosthesis (1) in particular has notches and/or flats as a contact surface for a turning tool.

9. The prosthesis as claimed in one of the preceding claims, **characterized in that** a horizontal projection of the crown (5) has the form of a circle or of a substantially uniform polygon and in particular that the free edge has a substantially cylindrical section (16).

10. The prosthesis as claimed in claim 9, **characterized in that** crown (5) is arranged substantially coaxially with respect to an edge of the spherical calotte (2).

11. The prosthesis as claimed in one of the preceding claims, **characterized in that** spherical calotte (2) and crown (5) are formed together in one piece and/or are welded to one another.

12. The prosthesis as claimed in one of the preceding claims, **characterized in that** spherical calotte (2) and crown (5) are designed as individual parts and are connected to one another by means of a mechanical fixing means, in particular by means of a screw thread, a bayonet connector or a clamping device.

13. A set of material for manufacturing a prosthesis as claimed in claim 12, **characterized in that** it comprises various spherical calottes (2) as claimed in claim 12 and various crowns (5) as claimed in claim 12, wherein the spherical calottes (2) and the crowns (5) can be fixed to one another in pairs in different combinations to create specially matched prostheses (1).

14. A set of prostheses as claimed in one of claims 1 to 12, which set is formed from prostheses having spherical calottes of different diameters but in particular the same ratio of the height h of the spherical calotte to the respective ball diameter, with which the diameter of the crown is in each case substantially the same percentage of the diameter of the spherical calotte.

## Revendications

1. Prothèse de remplacement superficiel au niveau d'une sphère d'articulations sphériques, la prothèse (1) comportant une calotte sphérique (2) qui présente une surface extérieure (3) conformée pour porter dans une cavité articulaire et comportant une couronne (5) qui est destinée à la fixation et/ou à la meilleure répartition des forces d'appui et qui divise une cavité, formée par la calotte sphérique (2) destinée à recevoir une extrémité osseuse, sensiblement en une première cavité (13) et une seconde cavité (14), et la calotte sphérique (2) ayant sensiblement une forme de demi-sphère ou de préférence d'une portion d'une demi-sphère, **caractérisée en ce que** le bord libre de la couronne (5) s'étend sensiblement dans le même plan que le bord libre de la calotte sphérique (2).

2. Prothèse selon la revendication 1, **caractérisée en ce que** la calotte sphérique a une hauteur h qui a une valeur allant de 65% à 90% du rayon de la sphère et notamment de 70% à 85% et notamment d'environ 80% du rayon de la sphère.

3. Prothèse selon l'une des revendications précédentes, **caractérisée en ce qu'**un diamètre de la couronne (5) a une valeur allant de 45 à 75%, notamment 55 à 65% et notamment d'environ 60%, d'un diamètre de la calotte sphérique (2).

4. Prothèse selon l'une des revendications précédentes, **caractérisée en ce qu'**une surface intérieure (4) de la calotte sphérique (2) et/ou une surface de la couronne (5) est conformée en vue d'un contact et/ou d'une adhérence avec un os et est, à cet effet, notamment rugueuse.

5. Prothèse selon l'une des revendications précédentes, **caractérisée en ce que** la couronne (5) comporte au moins un évidement (9), notamment cinq à dix évidements (9).

6. Prothèse selon l'une des revendications précédentes, **caractérisée en ce que** la couronne (5) présente sur une surface extérieure (6) et/ou une surface intérieure (7) un relief, notamment une cannelure (8), qui est formé de renflements annulaires faisant le tour de la couronne (5).

7. Prothèse selon l'une des revendications précédentes, **caractérisée en ce que** la calotte sphérique (2) présente sur une surface intérieure (4) un relief, notamment une cannelure, qui s'étend le long d'un bord de la calotte sphérique (2).

8. Prothèse selon l'une des revendications précédentes, **caractérisée en ce qu'**elle est conformée pour être vissée et dévissée, la couronne (5) et/ou une surface intérieure (4) de la calotte sphérique (2) comportant un filetage et la prothèse (1) comportant notamment des rainures et/ou des méplats conformés en surface d'attaque d'un outil rotatif.

9. Prothèse selon l'une des revendications précédentes, **caractérisée en ce qu'**un tracé de la couronne (5) a une forme d'un cercle ou d'un polygone sensiblement régulier et notamment **en ce que** le bord libre comporte une portion sensiblement cylindrique (16).

10. Prothèse selon la revendication 9, **caractérisée en ce que** la couronne (5) est disposée de façon sensiblement coaxiale à un bord de la calotte sphérique (2).

11. Prothèse selon l'une des revendications précédentes, **caractérisée en ce que** la calotte sphérique (2) et la couronne (5) sont conformées tous les deux d'une seule pièce et/ou sont soudées l'une à l'autre.

12. Prothèse selon l'une des revendications précédentes, **caractérisée en ce que** la calotte sphérique (2) et la couronne (5) sont conformées en pièces individuelles et sont reliées l'une à l'autre par un moyen de fixation mécanique, notamment par un filetage, une liaison à baïonnette ou un dispositif de serrage.

13. Jeu d'équipements pour réaliser une prothèse selon la revendication 12, **caractérisé en ce qu'**il comporte différentes calottes sphériques (2) selon la revendication 12 et différentes couronnes (5) selon la revendication 12, les calottes sphériques (2) et les couronnes (5) pouvant être fixées l'une à l'autre par paires selon différentes combinaisons afin de réaliser des prothèses (1) spécialement adaptées.

14. Jeu de prothèses selon l'une des revendications 1 à 12, lequel jeu de prothèses est formé avec des calottes sphériques de différents diamètres, mais notamment avec un même rapport de la hauteur h de la calotte sphérique sur le diamètre de sphère respectif, pour lesquelles le diamètre de la couronne est à chaque fois sensiblement égal au même pourcentage du diamètre de la calotte sphérique.
